# EUROPEAN PATENT SPECIFICATION

(11) **EP 3 328 493 B1**
(45) Date of publication and mention of the grant of the patent: **08.04.2020**
(21) Application number: 16744773.9
(22) Date of filing: 28.07.2016
(51) Int. Cl.: A61N 7/00

(54) **HAIR GROWTH STIMULATION**
HAARWACHSTUMSSTIMULATION
STIMULATION DE LA CROISSANCE DES CHEVEUX

(30) Priority: 29.07.2015 EP 15178818
(43) Date of publication of application: 06.06.2018
(73) Proprietor: Koninklijke Philips N.V., 5656 AG Eindhoven (NL)
(72) Inventor: THUMMA, Kiran Kumar, 5656 AE Eindhoven (NL); BOURQUIN, Yannyk Parulian Julian, 5656 AE Eindhoven (NL)
(74) Representative: Steenbeek, Leonardus Johannes
(86) International application number: PCT/EP2016/068074
(87) International publication number: WO 2017/017218

(56) References cited:
- US-A1- 2011 269 693
- US-A1- 2013 073 001
- US-A1- 2014 276 247
- US-A1- 2015 196 639
- US-B1- 6 234 990

## Description

### FIELD OF THE INVENTION

The invention relates to hair growth stimulation.

### BACKGROUND OF THE INVENTION

Hairs at different body locations have specific length. The length of the hairs is mainly define by two factors which are the duration of the anagen phase (growing phase of the hair) and the growth rate. Most of the products in the market are topical based or light based solutions for stimulating hair growth. There are many patents relating to hair growth and hardly any on making the existing hairs grow faster.

US2011230793 discloses a method for the treatment of alopecia and in particular the problem of male-pattern baldness. By application of controlled ultrasound exposures to specific regions in the skin tissue, a physiological response will develop which causes cells in the hair follicle to increase in number, and/or new follicles to form or multiply in response to controlled damage to existing cells, leading to new hair follicles and/or increased shaft thickness in existing hair follicles resulting in more robust hair growth. Preferably, the applicator utilizes a transducer capable of delivering high intensity ultrasound pulses of varying pulse lengths ranging from microseconds to milliseconds, with acoustic intensities at the focus ranging from a few thousand to tens of thousands of W/cm², and with acoustic frequencies ranging from a few MHz to approximately 20 MHz.

DE102008015486 discloses a hair device for stimulating hair growth that uses brush elements fed with ultrasound (i.e. beyond 20 kHz) to transfer vibrations onto the skin of a user's head. The scalp is stimulated so that blood circulation is enhanced and the penetration of active ingredients is promoted through the epidermis to the hair roots.

US 2011/0269693 discloses ultrasound delivery apparatus comprising flexible arrays of transducers and to methods and topical compositions for the treatment of skin, in particular for the treatment of cosmetic skin conditions and to improve the appearance of sun damaged and/or aged skin. The composition may comprise one or more anti-glycation agent, one or more antioxidant, a dermatologically acceptable excipient and optionally one or more substance capable of inducing expression of a molecular chaperone.

### SUMMARY OF THE INVENTION

It is, inter alia, an object of the invention to provide an improved hair growth stimulation without using chemicals such as the active ingredients and/or topical compositions known from some prior art documents. The invention is defined by the independent claim. Advantageous embodiments are defined in the dependent claims. The disclosure provides a hair growth stimulating device comprising an ultrasound transducer, and a driver for the ultrasound transducer, wherein the driver is arranged for having the ultrasound transducer generate an ultrasound signal having a frequency not exceeding 100 kHz, preferably 20 kHz, at a power not exceeding 500 mW/cm², preferably 250 mW/cm². The frequency may be subject to a frequency sweep not exceeding 20%, preferably 10%. Pulses having a pulse width between 10 ms and 500 ms may be used.

These and other aspects of the invention will be apparent from and elucidated with reference to the embodiments described hereinafter.

### BRIEF DESCRIPTION OF THE DRAWINGS

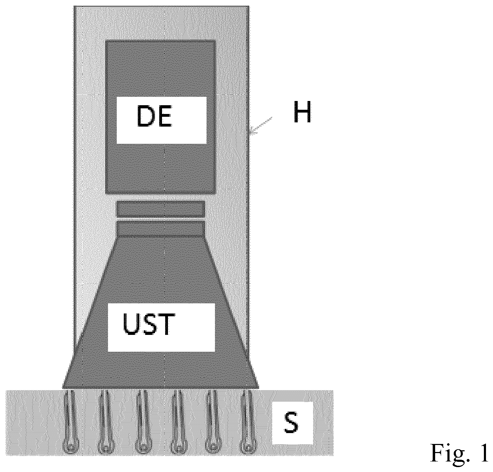

Fig. 1 shows an example of a hair growth stimulating device.

### DESCRIPTION OF EMBODIMENTS

Embodiments of the invention provide an ultrasonic treatment of hair whereby a larger area of skin/hair can be treated in order to make the hairs grow faster, whereby the total treatment time can be decreased. Faster hair growth by means of ultrasound using the parameters described within this invention is done not by heating the hair follicles but by non-thermal mechanisms which are the induced chemical/biological reactions by application of ultrasound. This removes the heating effects on the skin surface and also on the hair follicles.

Experiments have been done on ex-vivo skin with hair follicles. After the ultrasound treatment, the hairs are carefully isolated and cultured to see the growth of the hairs by taking the pictures every day. After 5 days of culturing the treated hairs, the hairs remain healthy. Moreover, the treated hairs grow 20%-30% faster than the untreated hairs. Statistical analysis shows significant difference between control and treated hairs. The ultrasound stimulates the faster hair growth by a direct stimulation of cells resulting in an increase of proliferation.

Fig. 1 shows an example of a hair growth stimulating device. A housing H comprises driver electronics DE and an ultrasound transducer UST, which are used on skin S having a plurality of hair follicles.

The device can be a single transducer which can be applied on the skin or in another embodiment, it can be an array of transducers. The array - which can treat a larger area - is made possible because of the limited heating of the skin using the parameters in accordance with the present disclosure.

Many settings have been tried in the lab on ex-vivo skin with grey hairs and black hairs. The desired faster hair growth can be achieved with the parameters given in the following table:

| | |
|---|---|
| Frequency | 20 kHz |
| Sweep bandwidth | 2 kHz |
| Sweep time | 1 s |
| Power | 100 mW/cm² - 500 mW/cm² |
| Treatment time | 10 s - 1 minute |

Using these parameters, we have measured the temperature increase at a few millimeters below the skin where the hair follicles are present. The temperature increase was between 3 - 5 °C for a treatment time of 1 minute. Such limited temperature increase will not result in discomfort for the user of the device. Additionally, this has been confirmed from simulations where we simulated the experimental setup with a transducer and the skin and calculated the temperature rise. The simulations showed an increase in temperature of 3 °C for a treatment time of 1 minute.

Further experiments were carried out at lower power levels of 500 mW/cm² and 250 mW/cm² using the otherwise the same parameters as described in table 2. The hair growth is faster at 250 mW/cm² than at 500 mW/cm². Above 1 W/cm² at a treatment time of above 10 s, no significant faster hair growth is measured.

The disclosure thus provides an ultrasonic hair stimulation device with specific advantages of large treatment area on different skin and hair types with favorable power levels and short treatment times. In an embodiment, specific frequencies (20-22 kHz with sweep) and power (between 100 - 500 mW/cm2) and a treatment time of 1 min are used. A pulse width of 10 ms or higher is used. Advantageously, no treatment medium is used.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The mere fact that certain measures are recited in mutually different dependent claims does not indicate that a combination of these measures cannot be used to advantage.

## Claims

1. Hair growth stimulating method to obtain a non-chemical hair growth stimulation, the hair growth stimulating method comprising:
generating an ultrasound signal having a frequency not exceeding 100 kHz at a power density not exceeding 500 mW/cm², and
applying the ultrasound signal to hair follicles.

2. Hair growth stimulating method as claimed in claim 1, wherein the frequency is between 20 and 30 kHz, and preferably between 20 and 25 kHz, more preferably 20 kHz.

3. Hair growth stimulating method as claimed in claim 1 or 2, wherein the frequency is subject to a frequency sweep not exceeding 20%, preferably 10%.

4. Hair growth stimulating method as claimed in any of the preceding claims 1-3, wherein pulses having a pulse width between 10 ms and 500 ms are used.

5. Hair growth stimulating method as claimed in any of the preceding claims 1-4, wherein the power density is between 100 - 500 mW/cm², and preferably 250 mW/cm².

6. Hair growth stimulating method as claimed in any of the preceding claims 1-5, wherein ultrasound is applied during a period between 0.5 and 2 minutes, and preferably during about 1 minute.

## Patentansprüche

1. Haarwuchsstimulationsverfahren zum Erhalten einer nicht-chemischen Haarwuchsstimulation, wobei das Haarwuchsstimulationsverfahren Folgendes umfasst:
Erzeugen eines Ultraschallsignals, das eine Frequenz aufweist, die 100 kHz bei einer Leistungsdichte, die 500 mW/cm² nicht überschreitet, nicht überschreitet, und
Anwenden des Ultraschallsignals an Haarfollikel.

2. Haarwuchsstimulationsverfahren nach Anspruch 1, wobei die Frequenz zwischen 20 und 30 kHz und vorzugsweise zwischen 20 und 25 kHz liegt, bevorzugter 20 kHz beträgt.

3. Haarwuchsstimulationsverfahren nach Anspruch 1 oder 2, wobei die Frequenz einem Frequenzhub, der 20 %, vorzugsweise 10 % nicht überschreitet, unterliegt.

4. Haarwuchsstimulationsverfahren nach einem der Ansprüche 1 bis 3, wobei Impulse, die eine Impulsbreite zwischen 10 ms und 500 ms aufweisen, verwendet werden.

5. Haarwuchsstimulationsverfahren nach einem der Ansprüche 1 bis 4, wobei die Leistungsdichte zwischen 100 und 500 mW/cm² liegt und vorzugsweise 250 mW/cm² beträgt.

6. Haarwuchsstimulationsverfahren nach einem der Ansprüche 1 bis 5, wobei Ultraschall während einer Periode zwischen 0,5 und 2 Minuten und vorzugsweise während etwa einer Minute angewandt wird.

## Revendications

1. Procédé de stimulation de la pousse des cheveux pour obtenir une stimulation de la pousse des cheveux non-chimique, le procédé de stimulation de la pousse des cheveux comprenant :
la génération d'un signal ultrason présentant une fréquence ne dépassant pas 100 kHz à une densité de puissance ne dépassant pas 500 mW/cm², et
l'application du signal ultrason à des follicules de cheveux.

2. Procédé de stimulation de la pousse des cheveux selon la revendication 1, dans lequel la fréquence est entre 20 et 30 kHz, et de préférence entre 20 et 25 kHz, plus préférentiellement 20 kHz.

3. Procédé de stimulation de la pousse des cheveux selon la revendication 1 ou 2, dans lequel la fréquence est sujette à un balayage de fréquence ne dépassant pas 20 %, de préférence 10 %.

4. Procédé de stimulation de la pousse des cheveux selon l'une quelconque des revendications 1 à 3, dans lequel les impulsions présentant une largeur d'impulsion entre 10 ms et 500 ms sont utilisées.

5. Procédé de stimulation de la pousse des cheveux selon l'une quelconque des revendications précédentes 1 à 4, dans lequel la densité de puissance est entre 100 et 500 mW/cm² et de préférence 250 mW/cm².

6. Procédé de stimulation de la pousse des cheveux selon l'une quelconque des revendications précédentes 1 à 5, dans lequel un ultrason est appliqué durant une période entre 0,5 et 2 minutes, et de préférence pendant environ 1 minute.
